(19) Europäisches Patentamt
European Patent Office
Office européen des brevets

(11) Veröffentlichungsnummer: **0 044 931 B1**

# (12) EUROPÄISCHE PATENTSCHRIFT

(45) Veröffentlichungstag der Patentschrift: **10.04.85**

(21) Anmeldenummer: **81104568.1**

(22) Anmeldetag: **13.06.81**

(51) Int. Cl.⁴: **C 07 D 493/04**, A 61 K 31/34, A 61 K 31/455 // (C07D493/04, 307:00, 307:00)

(54) **2-0- und 5-0-substituierte 1.4;3.6-Dianhydro-hexit-mononitrate, Verfahren zu ihrer Herstellung und pharmazeutische Zubereitung.**

(30) Priorität: **25.07.80 DE 3028289**

(43) Veröffentlichungstag der Anmeldung: **03.02.82 Patentblatt 82/5**

(45) Bekanntmachung des Hinweises auf die Patenterteilung: **10.04.85 Patentblatt 85/15**

(84) Benannte Vertragsstaaten: **AT BE CH FR GB IT LI NL SE**

(56) Entgegenhaltungen:
**DE - A - 1 445 417**
**DE - B - 2 903 927**
**US - A - 3 886 186**
**US - A - 4 169 152**

**Die Akte enthält technische Angaben, die nach dem Eingang der Anmeldung eingereicht wurden und die nicht in dieser Patentschrift enthalten sind.**

(73) Patentinhaber: **Dr. Willmar Schwabe GmbH & Co., Willmar-Schwabe-Strasse 4, D-7500 Karlsruhe 41 (DE)**

(72) Erfinder: **Klessing, Klaus, Dr. Dipl.-Chem., Rheingoldstrasse 3, D-7505 Ettlingen 5 (DE)**
Erfinder: **Chatterjee, Shyam Sunder, Dr. Dipl.-Chem., Werrabronner Strasse 8a, D-7500 Karlsruhe 41 (DE)**

(74) Vertreter: **Bunke, Holger, Dr. Dipl.-Chem. et al, Patentanwälte Prinz, Bunke & Partner Lessingstrasse 9, D-7000 Stuttgart 1 (DE)**

**EP 0 044 931 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft 2-O- und 5-O-substituierte 1.4;3.6-Dianhydro-hexit-mononitrate der allgemeinen Formel I,

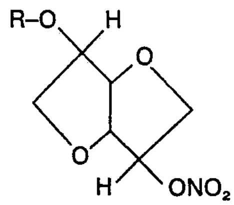

(I)

worin R eine halogensubstituierte Phenylgruppe, eine Methansulfonyl- oder Nicotinoylgruppe ist, sowie deren physiologisch unbedenkliche Säureadditionssalze, sofern R die Nicotinoylgruppe bedeutet.

Das Grundgerüst dieser Verbindungen besteht aus einem der stereoisomeren, unter Epimerisierung ineinander umwandelbaren 1.4;3.6-Dianhydro-hexite, nämlich entweder dem 1.4;3.6-Dianhydro-L-idit (= «Isoidid») (II),

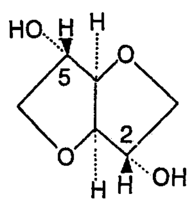

(II)

bei dem die OH-Gruppen in 2- und 5-Stellung jeweils exo-Konfiguration aufweisen, oder dem 1.4;3.6-Dianhydro-D-glucit (=«Isosorbid») (III),

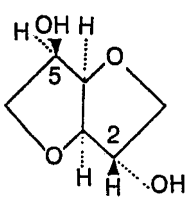

(III)

der eine 2-exo-ständige und eine 5-endo-ständige OH-Gruppe aufweist und somit – bei verschiedenen Substituenten in 2- und 5-Stellung in zwei isomeren Formen auftritt.

Das Grundgerüst einiger Verbindungen schliesslich besteht aus dem 1.4;3.6-Dianhydro-D-mannit (=«Isomannid») (IV),

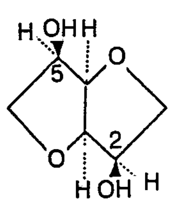

(IV)

der zwei endo-ständige OH-Gruppen aufweist.

Da im Gegensatz zu den Glucit-Derivaten bei den Idit- und Mannit-Derivaten eine Unterscheidung zwischen den 2- und 5-Substituenten nicht möglich ist, weil das $C^2$-Atom bei Drehung des Moleküls um 180°C zum $C^5$-Atom wird, sind Hinweise auf die 5-Stellung oder 2-Stellung von Substituenten bei diesen Derivaten an sich überflüssig. Des besseren Vergleiches der Strukturen der einzelnen Verbindungen mit den allgemeinen Formeln wegen werden aber hier die Isoidid-Derivate als in 5-O-Stellung substituierte Isoidid-Derivate bezeichnet, da sie durch nucleophile Substitution unter Konfigurationsumkehr beispielsweise aus 5-O-Alkylsulfonyl- oder 5-O-Arylsulfonyl-isosorbid hergestellt werden können.

Eine kurze Zusammenfassung über die Stereoisomerie der 1.4;3.6-Dianhydro-hexite gibt J.A. Mills in Advances in Carbohydrate Chem. 10, 1–53 (1955).

Die Erfindung betrifft weiter Verfahren zur Herstellung der eingangs genannten 2-O- und 5-O-substituierten 1.4;3.6-Dianhydro-hexit-mononitrate sowie pharmazeutische Zubereitungen, die die erfindungsgemässen Verbindungen enthalten.

Die Nitrate des 1.4;3.6-Dianhydro-D-glucits (auch 1.4;3.6-Dianhydro-D-sorbit genannt) sind z.B. aus der US-PS 3 886 186 bekannt, und zwar sowohl die 2- und 5-Mononitrate als auch das 2,5-Dinitrat des Isosorbids. Diese Nitrate, insbesondere das Dinitrat, das bereits als Arzneimittel im Handel ist, sind pharmakologisch wirksame Substanzen mit hämodynamischer, vasodilatorischer und antianginöser Wirksamkeit, die insbesondere bei Koronarinsuffizienz und zur Behandlung von Angina Pectoris verwendet werden.

Aus der US-PS 3 886 186 sind weiter das 5-Acetat-2-nitrat, 2-Acetat-5-nitrat-2-Äthylat-5-nitrat, 5-Nitrat-2-sulfamat, 2-Carbamat-5-nitrat und das 5-Carbamat-2-nitrat des Isosorbids bekannt sowie die entsprechenden Propionat-, Butyrat-, Isobutyrat-, Caproat- und Benzoat-mononitrate.

Ferner sind die p-Tuluolsulfonsäureester der Mononitrate von Isoidid, Isosorbid und Isomannid bekannt (Can. J. Chem. 45, 2191–2194 (1967)). Allerdings sind bisher nur die Infrarot-Spektren dieser Verbindungen näher beschrieben worden. Über die pharmakologischen Eigenschaften der Verbindungen ist dagegen nichts bekannt geworden.

Die Pharmakokinetik des Dinitrats und der Mononitrate von Isosorbid, Isomannid und Isoidid wurde von Bogaert und Rosseel in Naunyn-Schmiedeberg's Arch. Pharmacol. 275, 339 (1972) beschrieben.

Es hat sich jedoch gezeigt, dass die Nitrate unangenehme Nebenwirkungen, insbesondere Kopfschmerzen, verursachen. Die Mononitrate sind ausserdem schlechter resorbierbar als etwa das Isosorbiddinitrat (ISDN). Es kommt hinzu, dass sich die Dinitrate des Isosorbids, Isomannids und Isoidids nur unter besonderen Vorsichtsmassnahmen herstellen und handhaben lassen, da sie explosiv sind.

Somit bestand ein Bedürfnis nach der Bereitstellung neuer pharmazeutischer Mittel mit gleichem Wirkungsspektrum, die jedoch die genannten Nachteile nicht aufweisen, und nach der Schaffung neuer 1.4;3.6-Dianhydro-hexit-mononitrate, die als wirksame Bestandteile solcher pharmazeutischer Mittel verwendet werden können.

Die der Erfindung zugrundeliegende Aufgabe besteht darin, das angeführte Bedürfnis zu befriedigen, die Lösung dieser Aufgabe darin, die erfindungsgemässen Stoffe bereitzustellen.

Gegenstand der Erfindung sind somit 5-O-substituierte 1.4;3.6-Dianhydro-L-idit-2-nitrate der allgemeinen Formel V,

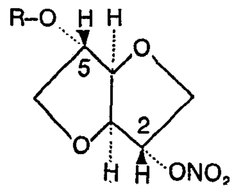

(V)

5-O-substituierte 1.4;3.6-Dianhydro-D-glucit-2-nitrate der allgemeinen Formel VI,

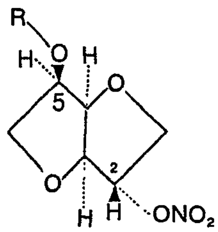

(VI)

2-O-substituierte 1.4;3.6-Dianhydro-D-glucit-5-nitrate der allgemeinen Formel VII,

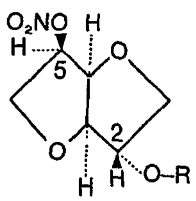

(VII)

sowie 5-O-substituierte 1.4.-3.6-Dianhydro-D-mannit-2-nitrate der allgemeinen Formel VIII,

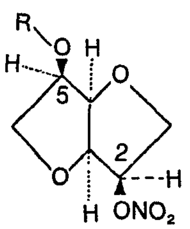

(VIII)

worin R jeweils entweder eine halogensubstituierte Phenylgruppe, eine Methansulfonyl- oder eine Nicotinoylgruppe bedeutet, sowie deren physiologisch unbedenkliche Säureadditionssalze, sofern R die Nicotinoylgruppe bedeutet.

Die erfindungsgemässen Verbindungen besitzen koronardurchflusssteigernde, spasmolytische, blutdrucksenkende, negativ inotrope und die Herzfrequenz senkende Wirksamkeit. Sie sind zur Behandlung von Koronarerkrankungen, zur Kupierung und Prophylaxe von Angina Pectoris-Anfällen, zur Nachbehandlung von Herzinfarkten und zur Behandlung von Herzinsuffizienzen geeignet. Die neuen Verbindungen besitzen eine gute therapeutische Breite. Die orale Absorption ist besonders gut und die Wirkungsdauer lang. Darüber hinaus bewirken sie eine Verbesserung der peripheren Durchblutung und der Gehirndurchblutung.

Die Handhabung und Herstellung der erfindungsgemässen Verbindungen ist weit weniger gefährlich als etwa beim bekannten ISDN, weil sie nicht explosiv sind.

Die erfindungsgemässen Verbindungen besitzen im 1.4;3.6-Dianhydro-hexit-Grundgerüst 4 asymmetrische C-Atome und liegen in optisch aktiver Form vor, da als Ausgangsverbindungen optisch reine 1.4;3.6-Dianhydro-hexite verwendet werden, die aus natürlich vorkommenden Zuckeralkoholen leicht zugänglich sind.

Die erfindungsgemässen Verbindungen können ausgehend von den epimeren, unsubstituierten 1.4;3.6-Dianhydro-hexiten, also ausgehend von L-Isoidid, D-Isosorbid und D-Isomannid, hergestellt werden, und zwar auf verschiedenen Synthesewegen:

Der erste erfindungsgemässe Weg zur Herstellung derjenigen erfindungsgemässen Verbindungen der allgemeinen Formel I, worin R den Methansulfonyl- oder Nicotinoyl-Rest bedeutet, besteht darin, dass der entsprechende 1.4;3.6-Dianhydrohexit mit Salpetersäure, Nitriersäure oder mit einem Gemisch aus Salpetersäure und Acetanhydrid/Essigsäure, gegebenenfalls in Gegenwart von Harnstoff, verestert wird, wobei jeweils ein Gemisch aus dem oder den entsprechenden Mononitraten und dem Dinitrat entsteht. Um den Anteil an Dinitrat möglichst niedrig zu halten, wählt man dabei das Verhältnis zwischen Salpetersäure und Isohexid vorteilhaft so, dass auf 1 Mol Isohexid 0,5 bis 1 Mol Salpetersäure eingesetzt werden. Das Gemisch der Mono- und Dinitrate wird in Äther aufgenommen und mit Wasser extrahiert, wobei das Mononitrat aus der wässrigen Phase isoliert werden kann, während das unerwünschte Dinitrat vollständig in der Ätherphase verbleibt. Im Falle der Veresterung zu den Isosorbid-nitraten ist es vorteilhaft, die Auftrennung der Reaktionsprodukte durch Säulenchromatographie an Kieselgel durchzuführen, wobei nicht nur das Dinitrat vollständig abgetrennt wird, sondern auch eine Auftrennung der beiden Mononitrate, nämlich des Isosorbid-5(endo)-nitrats und des Isosorbid-2(exo)-nitrats erfolgt.

Das entsprechende 1.4;3.6-Dianhydro-hexitmononitrat wird anschliessend mit dem gewünschten Säurechlorid (Methansulfonsäurechlorid oder Nicotinsäurechlorid) zu der gewünschten Verbindung umgesetzt. Nach diesem ersten Verfahren wird beispielsweise 1.4;3.6-Dianhydro-L-idit mit Salpetersäure verestert und das entstehende 1.4;3.6-Dianhydro-L-idit-2-nitrat mit Nicotinsäurechlorid zum 1.4;3.6-Dianhydro-L-idit-2-nitrat-5-nicotinat umgesetzt.

Das zweite erfindungsgemässe Verfahren zur Herstellung derjenigen Verbindungen der allgemeinen Formel I, worin R den Methansulfonyl- oder den Nicotinoylrest bedeutet, besteht darin, dass der entsprechende 1.4;3.6-Dianhydrohexit zuerst mit dem gewünschten Säurechlorid (Methansulfonsäurechlorid oder Nicotinsäurechlorid)

umgesetzt wird, wobei jeweils ein Gemisch aus den entsprechenden Di- und Monoestern entsteht, welches durch fraktionierte Kristallisation, fraktionierte Extraktion oder mit Hilfe anderer an sich bekannter Methoden aufgetrennt wird. Das gewünschte Monoacylat wird anschliessend mit Salpetersäure zu der gewünschten Verbindung verestert.

Nach diesem zweiten erfindungsgemässen Verfahren wird beispielsweise ausgehend von 1.4;3.6-Dianhydro-D-glucit durch Umsetzung mit Methansulfonsäurechlorid zunächst 1.4;3.6-Dianhydro-D-glucit-5-methansulfonat hergestellt und letzteres mit Salpetersäure, Nitriersäure oder mit einem Gemisch aus Salpetersäure, Eisessig und Acetanhydrid zum 1.4;3.6-Dianhydro-D-glucit-2-nitrat-5-methansulfonat umgesetzt.

Zur Herstellung derjenigen erfindungsgemässen Verbindungen der allgemeinen Formel I, worin R den Halogenphenyl-Rest bedeutet, stellt man zunächst ein reaktives Isohexidmonoacylat her, vorzugsweise – entsprechend dem vorstehend beschriebenen Verfahren – ein Isohexid-monomethansulfonat oder -toluolsulfonat, und setzt dieses mit einem geeigneten Salz des gewünschten Halogenphenols, beispielsweise mit p-Chlorphenol-Natriumsalz, das auch im Verlauf der Reaktion in situ durch Zugabe eines geeigneten Protonenfängers zu dem entsprechenden Halogenphenol erzeugt werden kann, beispielsweise durch Zugabe von Natriumhydrid, zu dem gewünschten Halogenphenyläther um. Der so hergestellte Halogenphenyläther des Isohexids wird anschliessend, wie zuvor beschrieben, mit Salpetersäure zum Mononitrat verestert, vorteilhaft bei Temperaturen unter 0 °C, um eine Nitrierung des Halogenphenylrestes zu vermeiden.

Die Verätherung wird vorzugsweise in einem dipolaren aprotischen Lösungsmittel, z.B. in Dimethylsulfoxid oder in Glykoldiäthern, bei erhöhter Temperatur durchgeführt und erfolgt nach dem Reaktionsmuster einer typischen bimolekularen nucleophilen Substitution ($S_N2$-Reaktion), die stets mit einer Umkehr der Konfiguration am zentralen Kohlenstoffatom verbunden ist. Diese Konfigurationsumkehr, dem Fachmann auch unter den Begriffen «Inversion» oder «Walden-Umkehr» geläufig, ist der Grund dafür, dass aus dem 1.4;3.6-Dianhydro-D-glucit-5-acyl-Derivat, bei dem der Acylrest in 5-Stellung endo-ständig vorliegt, stets das entsprechend verätherte 1.4;3.6-Dianhydro-L-idit-Derivat entsteht, bei dem die neu eingeführte Halogenphenyläther-Gruppe nicht mehr in endo-, sondern in exo-Stellung steht. Die mit der $S_N2$-Reaktion verbundene Walden-Umkehr ist in ganz entsprechender Weise verantwortlich dafür, dass aus dem entsprechenden 1.4;3.6-Dianhydro-L-iditacylat der in 5-Stellung endo-substituierte 1.4;3.6-Dianhydro-D-glucit-Äther, aus dem 1.4;3.6-Dianhydro-D-mannit-acylat der entsprechende, in 2-Stellung exo-substituierte 1.4;3.6-Dianhydro-D-glucit-Äther und aus dem 1.4;3.6-Dianhydro-D-glucit-2-exo-acylat der entsprechende, in 2-Stellung endosubstituierte 1.4;3.6-Dianhydro-D-mannit-Äther entsteht.

Entsprechend dem dritten erfindungsgemässen Verfahren können auch diejenigen erfindungsgemässen Verbindungen der allgemeinen Formeln V und VII hergestellt werden, worin R den Nicotinoylrest bedeutet und wobei der Nicotinsäurerest die 2(exo)- bzw. 5(exo)-Position des entsprechenden Isosorbid- bzw. Isoidid-nitrats einnimmt, indem man die entsprechenden Isohexid-methansulfonate mit endoständiger Methansulfonylgruppe der nucleophilen $X_N2$-Substitution durch geeignete Salze der Nicotinsäure, beispielsweise Natriumnicotinat, in einem dipolaren aprotischen Lösungsmittel, beispielsweise Dimethylformamid, bei erhöhter Temperatur unterwirft. Auch hier tritt Konfigurationsumkehr am Substitutionsort auf, jedoch ist die nucleophile Kraft des Nicotinat-Anions nicht mehr ausreichend, um auch die sterisch stärker gehinderten Isohexid-exo-methansulfonate zu substituieren.

Die so hergestellten Isohexid-nicotinate werden – wie zuvor beschrieben – mit Salpetersäure zu den Isohexid-nicotinat-nitraten verestert. Auf diese Weise kann beispielsweise das 1.4;3.6-Dianhydro-D-glucit-5-methansulfonat mit Natriumnicotinat in Dimethylformamid bei 150 °C zum 1.4;3.6-Dianhydro-L-idit-5-nicotinat umgesetzt werden, das anschliessend mit Salpetersäure mit 1.4;3.6-Dianhydro-L-idit-5-nicotinat-2-nitrat verestert wird.

Um diejenigen erfindungsgemässen Verbindungen der allgemeinen Formel I, worin R den Nicotinoylrest bedeutet, in ihre physiologisch unbedenklichen Salze überzuführen, können anorganische Säuren und Mineralsäuren wie Halogenwasserstoffsäuren und Phosphorsäuren sowie organische Säuren wie Carbon- und Sulfonsäure, beispielsweise Malon-, Bernstein-, Milch-, Wein-, Äpfel-, Benzoe-, Salicyl-, Citronen-, Ascorbin-, Nicotin- oder p-Toluolsulfonsäure verwendet werden. Die freien Basen können aus den Säureadditionssalzen wieder durch vorsichtige Behandlung mit Basen, beispielsweise Natrium- oder Kaliumhydrogencarbonat, freigesetzt werden.

Gegenstand der Erfindung sind ferner pharmazeutische Zubereitungen, die neben üblichen Träger- und Zusatzstoffen mindestens eine der erfindungsgemässen Verbindungen oder ihrer physiologisch unbedenklichen Salze enthalten. Diese Zubereitungen können als Arzneimittel in der Human- und Veterinärmedizin verwendet werden. Übliche Trägerstoffe sind beispielsweise Wasser, pflanzliche Öle, Polyäthylenglykole, Glycerinester, Gelatine, Kohlenhydrate wie Laktose oder Stärke, Magnesiumstearat, Talk, Vaseline. Übliche Zusatzstoffe sind beispielsweise Konservierungs-, Stabilisierungs-, Gleit-, Netzmittel, Emulgatoren, physiologisch unbedenkliche Salze, Puffersubstanzen, Farb-, Geschmacks- und Aromastoffe. Die Auswahl der Träger- und Zusatzstoffe hängt davon ab, ob die erfindungsgemässen Verbindungen enteral, parenteral oder topikal appliziert werden sollen.

Die erfindungsgemässen Verbindungen können auch im Gemisch mit anderen Wirkstoffen, beispielsweise Vitaminen oder bekannten, im Handel

befindlichen Herz-Kreislauf-Mitteln, insbesondere auch mit β-Rezeptorenblockern, verabreicht werden.

Beispiel für eine pharmezeutische Zubereitung

Für die Herstellung von Tabletten von je 100 mg Einzelgewicht, die je 5 mg Wirkstoff enthalten, benötigt man

I. 5 g 1.4;3.6-Dianhydro-D-glucit-2-nitrat-5-nicotinat
II. 54 g mikrokristalline Cellulose
III. 20 g Milchzucker
IV. 20 g Maisstärke
V. 0,5 g kolloidale Kieselsäure
VI. 0,5 g Magnesiumsteärat

Die Substanzen I–IV werden trocken 10 Min. lang gemischt, anschliessend wird das Gemisch der Substanzen V und VI zugegeben, man mischt weitere 10 Minuten und verpresst das so erhaltene Pulver auf einer Tablettiermaschine zu Tabletten von 100 mg Einzelgewicht.

Jeder der in den nachfolgenden Beispielen genannten erfindungsgemässen Verbindungen und Zwischenprodukte stellt ein zur Herstellung pharmazeutischer Zubereitungen besonders geeignetes Mittel dar.

Die in den Beispielen enthaltenen Abkürzungen haben folgende Bedeutungen:

Schmp. = Schmelzpunkt (unkorrigiert)
(Z) = Zersetzung
d = Dichte
$[\alpha]^{25}_D$ = optische Drehung bei 25 °C, Natrium-D-Linie.

Hinter den optischen Drehwerten sind die Konzentrationen der gemessenen Lösungen angegeben, wobei c 2 beispielsweise eine Konzentration von 1 g/100 ml Lösung bedeutet; das Lösungsmittel ist jeweils gesondert angegeben.

Alle Temperaturen sind in °C angegeben.

Beispiel Nr. 1

5-O-(4-Chlorphenyl)-1.4;3.6-dianhydro-L-idit-2-nitrat:

a) 1.4;3.6-Dianhydro-D-glucit-2-methansulfonat, -5-methansulfonat und -2.5-dimethansulfonat:

Zur Lösung von 4,82 kg (33 Mol) 1.4;3.6-Dianhydro-D-glucit in 24 Liter Pyridin tropt man unter Feuchtigkeitsausschluss, Rühren und Kühlen auf −15° bis −20° innerhalb mehrerer Stunden 3,1 Liter (40 Mol) Methansulfonsäurechlorid. Anschliessend rührt man 15 Stunden ohne Kühlung weiter. Man destilliert im Vak. das Pyridin ab, gibt zum öligen Rückstand 15 Liter Wasser, kocht auf und lässt abkühlen. Absaugen, Waschen mit 4 Litern Wasser und Trocknen des kristallinen Niederschlages ergibt 2,22 kg (7,34 Mol) 1.4;3.6-Dianhydro-D-glucit-2.5-dimethansulfonat. Das Filtrat wird unter Rühren und Wasserkühlung mit ca. 1,5 kg Natriumhydroxid neutralisiert und bei ca. 70° im Vakuum bis zur Trockne eingedampft. Der Trockenrückstand wird mit insgesamt 30 Litern Chloroform kontinuierlich heiss extrahiert und der Extrakt heiss filtriert. Man lässt den Extrakt 15 Stunden bei 20° stehen, saugt den kristallinen Niederschlag ab, wäscht ihn zweimal mit je 2 Litern Chloroform, trocknet und erhält 2,3 kg (10,26 Mol) 1.4;3.6-Dianhydro-D-glucit-5-methansulfonat. Die vereinigten Filtrate werden im Vakuum eingedampft und der Rückstand heiss in 22 Litern Äthanol gelöst. Man lässt 15 Stunden bei 20° stehen, saugt den kristallinen Niederschlag ab, wäscht ihn zweimal mit je 3 Litern Äthanol, trocknet und erhält 0,65 kg (2,90 Mol) 1.4;3.6-Dianhydro-D-glucit-2-methansulfonat. Eindampfen der Filtrate ergibt 2,21 kg (9,85 Mol) eines Gemisches der beiden isomeren Mono-methansulfonate, das je nach Bedarf durch Wiederholung der abwechselnden Kristallisationen aus Chloroform und Äthanol weiter aufgetrennt werden kann bzw. durch Veresterung mit Methansulfonsäurechlorid in Pyridin vollständig in 1.4;3.6-Dianhydro-D-glucit-2.5-dimethansulfonat übergeführt wird.

Analytische Mengen der Methansulfonate ergeben nach Umkristallisation korrekte Elementaranalysen und die in Tabelle 1 aufgeführten Schmelzpunkte und optische Drehungen:

Tabelle 1

| 1.4;3.6-Dianhydro-D-glucit | Umkristallisiert aus | Schmp. [°C] | $[\alpha]^{25}_D$ |
|---|---|---|---|
| -2-methansulfonat | Chloroform | 135–138.5 | 62.5 (c 2; Aceton) |
| -5-methansulfonat | Chloroform | 123–124 | 75.9 (c 2; Methanol) |
| -2.5-dimethansulfonat | Äthanol/ Aceton | 127–128 | 74 (c 2; Aceton) |

Anmerkung:
Setzt man 1.4;3.6-Dianhydro-D-glucit mit der 2 bis 2.5-fach molaren Menge Methansulfonsäurechlorid unter den gleichen Reaktionsbedingungen um, erhält man das 1.4;3.6-Dianhydro-D-glucit-2.5-dimethansulfonat in nahezu quantitativer Ausbeute.

b) 5-O-(4-Chlorphenyl)-1.4;3.6-dianhydro-L-idit:

Zu einer Lösung von 13,0 g 4-Chlorphenol (0,1 Mol) in 70 ml wasserfr. Dimethylsulfoxid (DMSO) werden portionenweise 2,4 g Natriumhydrid (0,1 Mol) gegeben. Unter Rühren und Feuchtigkeitsausschluss erwärmt man auf 80 °C und lässt eine Lösung von 11,2 g (0,05 Mol) 1.4;3.6-Dianhydro-D-glucit-5-methansulfonat in 30 ml wfr. DMSO zutropfen. Nach 24 Stdn. Rühren bei 80° kühlt man ab, gibt tropfenweise Wasser zur Hydrolyse von nicht umgesetztem Natriumhydrid und anschliessend 100 ml Wasser und 200 ml Chloroform zu. Man rührt gut durch, trennt die Chloroformphase ab, schüttelt sie nacheinander mit 200 ml Wasser, 100 ml 1molarer Natronlauge und 200 ml Wasser aus, trocknet über wasserfreiem Natriumsulfat und dampft im Vakuum ein. Umkristallisation des Rückstandes aus Chloroform/Äther ergibt 11,5 g (45 mmol) 5-O-(4-Chlorphenyl)-1.4;3.6-dianhydro-L-idit.

Schmp. 107–110°; $[\alpha]^{25}_D$ 17.0 (c 2; Chloroform).

Elementaranalyse: $C_{12}H_{13}ClO_4$ (256,68)

Ber.:

C (56,15, H (5,10), Cl (13,81)

Gef.:

C (55,97), H (5,07), Cl (13,9)

c) 5-O-(4-Chlorphenyl)-1.4;3.6-dianhydro-L-idit-1-nitrat:

Zur Lösung von 13 g (50 mmol) 5-O-(4-Chlorphenyl)-1.4;3.6-dianhydro-L-idit in 150 ml Essigsäure und 35 ml Acetanhydrid tropf man unter Rühren und Kühlen auf 10 °C die Lösung von 3,5 ml 65%iger Salpetersäure in 50 ml Eisessig und 12,5 ml Acetanhydrid innerhalb 1 Stde. und rührt über Nacht unter allmählicher Erwärmung auf Raumtemperatur nach. Man gibt je 250 ml Chloroform und Wasser zu, rührt durch, trennt die Chloroformphase ab, wäscht sie mit 250 ml Wasser und dampft sie nach dem Trocknen über wasserfr. Natriumsulfat unter reduziertem Druck ein. Umkristallisation des öligen Rückstands aus Äthanol ergibt 13,3 g (44 mmol) 5-O-(4-Chlorphenyl)-1.4;3.6-dianhydro-L-idit-2-nitrat.

Schmp. 76–77°; $[\alpha]^{25}_D$ 41,4 (c 2; Chloroform).

Elementaranalyse: $C_{12}H_{12}ClNO_6$ (301,69)

Ber.:

C (47,77), H (4,04), N (4,64), Cl (11,76)

Gef.:

C (47,97), H (4,03), N (4,38), Cl (11,9)

Beispiel Nr. 2

1.4;3.6-Dianhydro-L-idit-2-nitrat-5-nicotinat

a) 1.4;3.6-Dianhydro-L-idit-2-nitrat:

Zur Lösung von 62 g (0,42 Mol) 1.4;3.6-Dianhydro-L-idit in 800 ml Essigsäure und 280 ml Acetanhydrid tropft man unter Rühren und Kühlen auf 8 – 10° das Gemisch aus 18,5 ml (0,42 Mol) 95%iger Salpetersäure (d = 1,5), 400 ml Essigsäure und 100 ml Acetanhydrid und rührt 12 Stdn. nach. Man verdünnt mit 3,5 Litern Wasser, neutralisiert durch portionenweise Zugabe von Natriumhydrogencarbonat, extrahiert 5mal mit je 1000 ml Chloroform und engt die über wasserfr. Natriumsulfat getrockneten Chloroformextrakte unter reduziertem Druck bis fast zur Trockne ein. Das so erhaltene Gemisch aus Mono- und Dinitrat wird nach dem Lösen in 250 ml Äther 10mal mit je 100 ml Wasser extrahiert. Die Wasserphasen enthalten das Mononitrat (Rf = 0,47; Kieselgel-Fertigplatten, Chloroform/Methanol 9/l), in der Ätherphase verbleibt das Dinitrat (Rf = 0,75). Aus den Wasserphasen erhält man nach Sättigen mit Natriumchlorid, 5maliger Extraktion mit je 200 ml Chloroform und Eindampfen der Chloroformextrakte unter reduz. Druck 32,1 g (0,17 Mol) 1.4;3.6-Dianhydro-L-idit-2-nitrat in Form eines farblosen Öls, das ohne weitere Reinigung für die folgende Veresterung verwendet wird.

b) 1.4;3.6-Dianhydro-L-idit-2-nitrat-5-nicotinat:

Die Mischung aus 150 ml wasserfr. Pyridin, 9,5 g (50 mmol) 1.4.;3.6-Dianhydro-L-idit-2-nitrat und 7,7 g (55 mmol) Nicotinsäurechlorid wird 24 Stdn. unter Feuchtigkeitsausschluss bei 20° gerührt. Unter reduziertem Druck (10 mbar) dampft man das Pyridin ab, neutralisiert den Rückstand mit wässriger Natriumhydrogencarbonat-Lösung, dampft erneut unter reduziertem Druck ein und extrahiert den Rückstand mit siedendem Chloroform. Eindampfen des über wasserfreiem Natriumsulfat getrockneten Chloroformextrakts und Umkristallisation aus Methanol liefern 11,8 g (40 mmol) 1.4;3.6-Dianhydro-L-idit-2-nitrat-5-nicotinat.

Schmp. 81–3°; $[\alpha]^{25}_D$ 111 (c 2,0; Chloroform)

Elementaranalyse: $C_{12}H_{12}N_2O_7$ (296,24)

Ber.:

C (48,65), H (4,08), N (9,46)

Gef.:

C (48,85), H (4,15), N (9,42)

Beispiel Nr. 3

1.4;3.6-Dianhydro-L-idit-2-nitrat-5-nicotinat

a) 1.4;3.6-Dianhydro-L-idit-5-nicotinat:

Die Mischung aus 45 g (0,2 Mol) 1.4;3.6-Dianhydro-D-glucit-5-methansulfonat (hergestellt nach Beispiel 1a), 32 g (0,22 Mol) Natriumnicotinat und 500 ml wasserfr. Dimethylformamid wird 15 Stdn. bei 150 °C gerührt. Man destilliert das Lösungsmittel ab, löst den Rückstand in 200 ml Wasser, gibt 5 g Aktivkohle zu und filtriert. Das Filtrat wird mit verdünnter Natronlauge auf pH = 8–9 gestellt und 3mal mit je 200 ml Chloroform extrahiert. Die vereinigtenChloroformextrakte werden 3mal mit je 200 ml 0,25molarer Salzsäure extrahiert, die vereinigten salzsauren Extrakte nach dem Waschen mit 200 ml Chloroform unter reduziertem Druck eingedampft und mit Äthanol/Chloroform azeotrop getrocknet. Umkristallisation des Rückstands aus Äthanol/Isopropanol ergibt 17,4 g (60,5 mmol) kristallines 1.4;3.6-Dianhydro-L-idit-5-nicotinat-Hydrochlorid.

Schmp. 148–50°; $[\alpha]^{25}_D$ 52,6 (c 1,0; Wasser).

Elementaranalyse: $C_{12}H_{13}NO_5 \times HCl$ (287,70)

Ber.:

C (50,10), H (4,90), N (4,87), Cl (12,32)

Gef.:

C (50,03), H (4,88), N (4,87), Cl (11,8)

b) 1.4;3.6-Dianhydro-L-idit-2-nitrat-5-nicotinat:

Zur Lösung von 3,5 g (58 mmol) Harnstoff in 50 ml 95%iger Salpetersäure tropft man unter

Rühren und Kühlen auf –20° die Lösung von 14 g (56 mmol) 1.4;3.6-Dianhydro-L-idit-5-nicotinat (freie Base des zuvor gewonnenen Hydrochlorids) in 30 ml Methansulfonsäure, rührt 1 Std. bei –20° nach, giesst in 200 ml Eiswasser ein und tropft unter Kühlen 65 g Natriumhydroxid, gelöst in 200 ml Wasser, zu. Durch Zugabe von Natriumhydrogencarbonat vervollständigt man die Neutralisation und extrahiert 5mal mit je 100 ml Chloroform. Die vereinigten Extrakte ergeben nach dem Waschen mit 100 ml Wasser, Trocknen über wasserfr. Natriumsulfat und Eindampfen unter reduziertem Druck 10,8 g (36,5 mmol) 1.4;3.6-Dianhydro-L-idit-2-nitrat-5-nicotinat in Form eines farblosen langsam erstarrenden Öls. Man löst das Öl in Chloroform, leitet Chlorwasserstoff ein, fällt das Hydrochlorid mit Äther aus und kristallisiert aus Isopropanol/Äther um.

Schmp. 151–2° (Z); $[\alpha]^{25}_D$ 77,2 (c 0,5; Äthanol)

Elementaranalyse: $C_{12}H_{12}N_2O_7 \times HCl$ (332,69)

Ber.:

C (43,32), H (3,94), N (8,42), Cl (10,66)

Gef.:

C (43,55), H (3,91), N (8,40), Cl (10,1)

Beispiel Nr. 4

1.4;3.6-Dianhydro-D-glucit-2-nitrat-5-methansulfonat

Zur Lösung von 22,4 g (100 mmol) 1.4;3.6-Dianhydro-D-glucit-5-methansulfonat (hergestellt nach Beispiel 1a) in 200 ml Essigsäure und 75 ml Acetanhydrid tropf man unter Rühren und Kühlen auf 10° die Lösung von 4,5 ml (100 mmol) 95%iger Salpetersäure in 100 ml Essigsäure und 25 ml Acetanhydrid langsam zu, rührt unter allmählichem Erwärmen auf Raumtemperatur 4 Stdn. nach, gibt 1200 ml Wasser zu, neutralisiert portionenweise mit Natriumhydrogencarbonat und extrahiert 3mal mit je 300 ml Chloroform. Die Chloroformextrakte ergeben nach den Trocknen über wasserfr. Natriumsulfat, Eindampfen unter reduziertem Druck und Umkristallisation aus Chloroform/Äther 22,6 g (84 mmol) 1.4; 3.6-Dianhydro-D-glucit-2-nitrat 5-methansulfonat.

Schmp. 105–6°; $[\alpha]^{25}_D$ 91 (c 2,0; Chloroform)

Elementaranalyse: $C_7H_{11}NO_8S$ (269,24)

Ber.:

C (31,23), H (4,12), N (5,20), S (11,91)

Gef.:

C (31,62), H (4,20), N (5,06), S (12,0)

Beispiel Nr. 5

1.4;3.6-Dianhydro-D-glucit-2-nitrat-5-nicotinat

a) Veresterung von 1.4;3.6-Dianhydro-D-glucit mit Nicotinsäure:

Die Lösung von 73 g (500 mmol) 1.4;3.6-Dianhydro-D-glucit in 1500 ml wasserfr. Pyridin wird unter Rühren, Feuchtigkeitsausschluss und Kühlen auf 10° portionenweise mit insgesamt 72 g (500 mmol Nicotinsäurechlorid versetzt und bis zur vollständigen Umsetzung des Säurechlorids bei 50–60° gerührt (ca. 8 Stdn.). Unter vermindertem Druck destilliert man das Pyridin ab, stellt den Rückstand mit wässriger Natriumcarbonat-Lösung auf pH = 8 ein, dampft erneut unter reduziertem Druck ein und extrahiert den Rückstand 3mal mit je 400 ml siedendem Chloroform. Die vereinigten Chloroformextrakte werden auf 500 ml Volumen konzentriert und einer 40stufigen Craig'schen Gegenstromverteilung mit Wasser (+1% Äthanol) als mobile Oberphase unterworfen.

Die einzelnen Fraktionen werden dünnschichtchromatographisch (Kieselgel F 254 – Fertigplatten, Chloroform/Methanol 9/l) charakterisiert, entsprechend den Inhaltsstoffen vereinigt und eingedampft. Man erhält aus den

Fraktionen 1–3:

48,5 g (136 mmol) 1.4;3.6-Dianhydro-D-glucit-2.5-dinicotinat (Rf = 0,49). Schmp. nach Umkristallisation aus Chloroform/Äther: 142–4° (Lit.: Brit. Patent 1 027 891; Schmp. 143,5–144°); $[\alpha]^{25}_D$ 30,0 (c 2,0; Chloroform).

(Lit.: 28,8; Chloroform)

Elementaranalyse: $C_{18}H_{16}N_2O_6$ (356,34)

Ber.:

C (60,67), H (4,53), N (7,86)

Gef.:

C (60,82), H (4,55), N (7,84)

Fraktionen 4–14:

18,6 g (74 mmol 1.4;3.6-Dianhydro-D-glucit-2-nicotinat (Rf = 0,32). Schmp. nach Umkristallisation aus Chloroform/Petroläther 112–113,5°; $[\alpha]^{25}_D$ 69,8 (c 2,0; Chloroform).

Elementaranalyse: $C_{12}H_{13}NO_5$ (251,24)

Ber.:

C (57,37), H (5,21), N (5,58)

Gef.:

C (56,93), H (5,22), N (5,47)

Fraktionen 15–19:

4,5 g (18 mmol) Gemisch aus 1.4;3.6-dianhydro-D-glucit-2- und -5-nicotinat.

Fraktionen 20–36:

30,6 g (122 mmol) 1.4;3.6-Dianhydro-D-glucit-5-nicotinat (Rf = 0,25). Schmp. nach Umkristallisation aus Chloroform/Äther: 84–85,5°; $[\alpha]^{25}_D$ 55,4 (c 2,0; Chloroform).

Elementaranalyse: $C_{12}H_{13}NO_5$ (251,24)

Ber.:

C (57,37), H (5,21), N (5,58)

Gef.:

C (57,60), H (5,29), N (5,56)

Die Fraktionen 37–40 enthalten nicht umgesetzten 1.4;3.6-Dinahydro-D-glucit.

b) 1.4;3.6-Dianhydro-D-glucit-2-nitrat-5-nicotinat:

Der Lösung von 25 g (100 mmol) des zuvor erhaltenen 1.4;3.6-Dianhydro-D-glucit-5-nicotinats in 300 ml Essigsäure und 70 ml Acetanhydrid wird unter Rühren und Kühlen auf 10° die Lösung von 6,6 ml (150 mmol) 95%ig. Salpetersäure in 100 ml Essigsäure langsam zugetropft und 3 Stdn. bei dieser Temperatur nachgerührt. Man verdünnt mit 1500 ml Wasser, neutralisiert durch portionenweise Zugabe von Natriumhydrogencarbonat und extrahiert 4mal mit je 500 ml Äthylacetat. Die vereinigten Extrakte ergeben nach dem Waschen mit wässriger Natriumhydrogencarbonat-Lösung,

Trocknen über wasserfr. Natriumsulfat, Eindampfen unter reduziertem Druck und Umkristallisation aus Methanol/Wasser 23,7 g (80 mmol) 1.4;3.6-Dianhydro-D-glucit-2-nitrat-5-nicotinat.

Schmp. 107–108,5°; $[\alpha]_D^{25}$ 58,0 (c 2,0; Chloroform).

Elementaranalyse: $C_{12}H_{12}N_2O_7$ (296,24)

Ber.:

C (48,65), H (4,08), N (9,46)

Gef.:

C (48,55), H (3,98), N (9,47)

Beispiel Nr. 6

1.4;3.6-Dianhydro-D-glucit-2-nicotinat-5-nitrat:

12,5 g (50 mmol) des nach Beispiel 5 a) erhaltenen 1.4;3.6-Dianhydro-D-glucit-2-nicotinats werden analog Beispiel 5 b) mit Salpetersäure/Acetanhydrid/Essigsäure umsetzt und ergeben nach Umkristallisation aus Chloroform/Äther 10,7 g (36 mmol) 1.4;3.6-Dianhydro-D-glucit-2-nicotinat-5-nitrat.

Schmp. 54–7°; $[\alpha]^{25}_D$ 117,2 (c 2,0; Chloroform).

Elementaranalyse: $C_{12}H_{12}N_2O_7$ (296,24)

Ber.:

C (48,65), H (4,08), N (9,46)

Gef.:

C (48,73), H (4,11), N (9,40)

Beispiel Nr. 7

1.4,-3.6-Dianhydro-D-glucit-2-nicotinat-5-nitrat:

a) 1.4;3.6-Dianhydro-D-glucit-2- und -5-nitrat:

Der Lösung von 30 g (100 mmol) 1.4;3.6-Dianhydro-D-glucit in 300 ml Essigsäure und 70 ml Acetanhydrid tropft man unter Rühren und Kühlen auf 8° die Lösung von 7 ml (100 mmol) 65%iger Salpetersäure in 50 ml Essigsäure zu und rührt noch 4 Stdn. nach. Man verdünnt mit 1000 ml Wasser und extrahiert 3mal mit je 500 ml Äthylacetat. Die vereinigten Extrakte werden unter Rühren durch portionenweise Zugabe von wässriger Natriumhydrogencarbonat Lösung entsäuert. Man trennt die Äthylacetatphase ab, trocknet sie über wasserfreiem Natriumsulfat und engt unter reduziertem Druck auf ca. 50 ml ein. Das Konzentrat wird säulenchromatographisch über 1 kg Kieselgel mit Toluol/Äthylacetat 1/1 als Eluens aufgetrennt.

Die Fraktionen mit Rf = 0,26 (Kieselgel F 254 Fertigplatten; Toluol/Äthylacetat 1/1) liefern nach dem Eindampfen unter reduziertem Druck und Umkristallisation aus Äther 2,6 g (13,6 mmol) 1.4;3.6-Dianhydro-D-glucit-2-nitrat.

Schmp. 53–55,5° (Lit.: L. D. Hayward et al., Can. J. Chem. 45, 2191–4, 1967. Schmp. 52–3°); $[\alpha]^{25}_D$ 76,4 (c 2,0; Chloroform).

Elementaranalyse: $C_6H_9NO_6$ (191,15)

Ber.:

C (37,70), H (4,74), N (7,33)

Gef.:

C (37,83), H (4,85), N (7,25)

Die Fraktionen mit Rf = 0,20 ergeben nach dem Eindampfen unter reduz. Druck und Umkristallisation aus Äthylacetat/Petroläther 8,8 g (46 mmol) 1.4;3.6-Dianhydro-D-glucit-5-nitrat. Schmp. 86–88° (Lit.: 89,5–91°); $[\alpha]^{25}_D$ 163,4 (c 2,0; Chloroform).

Elementaranalyse: $C_6H_9NO_6$ (191,15)

Ber.:

C (37,70), H (4,74), N (7,33)

Gef.:

C (37,94), H (4,88), N (7,09)

b) 1.4;3.6-Dianhydro-D-glucit-2-nicotinat-5-nitrat:

7,65 g (40 mmol) des so erhaltenen 1.4;3.6-Dianhydro-D-glucit-5-nitrats werden analog Beispiel 2b mit Nicotinsäurechlorid in Pyridin umgesetzt und liefern nach Umkristallisation aus Chloroform/Äther 9,0 g (30,4 mmol) 1.4;3.6-Dianhydro-D-glucit-2-nicotinat-5-nitrat.

Schmp. 55–7°; $[\alpha]^{25}_D$ = 117 (c 2; Chloroform).

Beispiel Nr. 8

1.4;3.6-Dianhydro-D-mannit-2-nitrat-5-nicotinat:

Zur Lösung von 10,3 g (41 mmol) 1.4;3.6-Dianhydro-D-mannit-2-nicotinat in 100 ml Essigsäure gibt man 20 ml Acetanhydrid und 0,6 g Harnstoff, tropft unter Rühren und Kühlen auf 10 °C die Lösung von 8,8 ml (200 mmol) 96%iger Salpetersäure in 20 ml Essigsäure zu und lässt über Nacht bei Raumtemperatur weiterrühren. Man giesst dann unter Rühren in 500 ml Eiswasser, rührt ½ Std. nach und neutralisiert durch Zugabe von 500 ml 20%iger Natronlauge unter Kühlen und anschliessend durch Zugabe von Natriumhydrogencarbonat. Ausfallendes Produkt wird dabei durch Zufügen von etwas Chloroform in Lösung gebracht. Die Mischung wird 4mal mit je 200 ml Chloroform extrahiert; die vereinigten Chloroformextrakte ergeben nach dem Waschen mit 200 ml Wasser, Trocknen über wasserfreiem Natriumsulfat und Eindampfen unter reduziertem Druck öliges Rohprodukt, aus dem man nach Umkristallisation aus Isopropanol und anschliessend aus Äthanol 10,3 g (34,8 mmol) reines 1.4;3.6-Dianhydro-D-mannit-2-nitrat-5-nicotinat erhält.

Schmp. 132–134°; $[\alpha]^{20}_D$ 264,3 (c 0,5; Chloroform).

Elementaranalyse: $C_{12}N_{12}N_2O_7$ (296,24)

Ber.:

C (48,65), H (4,08), N (9,46)

Gef.:

C (48,70), H (4,27), N (9,59)

Als Vergleichsverbindungen wurden bei der Untersuchung der pharmakologischen Eigenschaften der erfindungsgemässen Verbindungen stets die im Handel erhältlichen Verbindungen Isosorbiddinitrat (ISDN) und Isosorbidmononitrat (ISMN) verwendet, wobei es sich bei ISMN um das 1.4;3.6-Dianhydro-D-glucit-2-nitrat handelt.

Die koronardurchflusssteigernde Wirksamkeit der erfindungsgemässen Verbindungen wurde an isolierten Meerschweinchenherzen (isolierte Herzen nach Langendorff, Methode nach Bunger et al.; Pflüger's Archiv 353, 317–325 (1975)) ermittelt. Nach Erreichen des stationären Zustands in der 30. Minute wurden die Herzen mit je 50 ml Tyrodelösung mit einem Gehalt an Prüfsubstanz von jeweils 25 µg/ml perfundiert. Jede Prüfsubstanz wurde an 3–6 Herzen geprüft.

Gemessen wurde jeweils die Inotropie, der Durchfluss und die Frequenz, wobei die in der Tabelle I angegebenen Werte Mittelwerte der prozentualen Änderungen gegenüber dem Vorlauf sind. Der Vergleich der gemessenen Werte zeigt, dass die koronardurchflusssteigernde Wirksamkeit der erfindungsgemässen Verbindungen grösser als diejenige von ISMN ist. Manche der erfindungsgemässen Verbindungen zeigen günstige inotrope und frequenzsenkende Wirkungen.

Die spasmolytische Wirksamkeit der erfindungsgemässen Verbindungen wurde an isolierten Ratten-Aorta-Präparaten mit Noradrenalin- und Kaliumchlorid-induzierten Kontaktionen bestimmt (Methode nach Wende und Peiper, Pflüger's Archiv 320, 1970, 133–141; und Towart und Stoepel, Naunyn Schmiedeberg's-Archives of Pharmacology; Suppl. Vol. 308, 1979, R 18). Als Vergleichsverbindungen wurden auch hier ISDN und ISMN gewählt.

In der Tabelle II sind die Konzentrationen (Molar) der Prüfsubstanzen eingetragen, die für 50% Hemmung des Spasmus notwendig sind ($ED_{50}$-Werte). Die spasmolytischen Wirksamkeiten der erfindungsgemässen Verbindungen sind besser als diejenige von ISMN und teilweise sind sie besser als diejenigen von ISDN.

Die blutdrucksenkende Wirksamkeit der erfindungsgemässen Verbindungen wurde im Vergleich zu ISDN und ISMN an narkotisierten Meerschweinchen nach i.v.-Gabe gemessen. Die in Tabelle III wiedergegebenen Werte zeigen, dass die erfindungsgemässen Verbindungen durchweg wirksamer als ISMN sind, wobei die Verbindungen Nr. 2b und 5b ähnliche Wirkungen wie ISDN besitzen.

Die inotrope und frequenzsenkende Herzkreislaufwirksamkeit der erfindungsgemässen Verbindung Nr. 5b wurde weiterhin an mischrassigen Katzen von 2,5–3,5 kg Körpergewicht bei intraduodenaler Applikation bestimmt. Die Tiere wurden mit einem Gemisch von Chloralose-Urethan narkotisiert (1,2 g/kg Urethan + 40 mg/kg Chloralose i.p. verabreicht). Sie atmeten spontan durch eine Tracheakanüle. Die A. Carotis sinistra wurde benutzt, um ein Katheter-Tip-Manometer in die linke Herzkammer zu legen. Die V. Jugularis diente zu Injektionszwecken. Über die A. Femoralis dextra wurde ein Katheter bis in die Aorta descendans vorgeschoben und an einen Druckaufnehmer (Statham P 23 Db) angeschlossen. Die Herzfrequenz wurde mit einem Pulsfrequenzmesser (Fa. Hugo Sachs Elektronik) aus dem linksventrikulären Drucksignal registriert. Durch eine Laparotomie, wurde eine Duodenalschlinge freigelegt. Die zu prüfenden Substanzen wurden direkt ins Lumen injiziert.

Wie sich aus den in der Tabelle IV angegebenen Werten ergibt, ist die blutdrucksenkende Wirkung der geprüften erfindungsgemässen Verbindung besser als diejenige der Vergleichsverbindung ISDN.

Tabelle I

Versuche am Langendorff-Herzen

Die in der Tabelle aufgeführten Werte zeigen die prozentuale Änderung gegenüber dem Vorlauf

| Substanz | Inotropie | Durchfluß | Frequenz |
|---|---|---|---|
| ISMN | + 5,40 | + 9,70 | +2,42 |
| 1c | +12,25 | +19,81 | −2,89 |
| 2b | + 3,36 | +35,37 | ±0 |
| 4 | ± 0 | +44,26 | ±0 |
| 5b | +15,77 | +23,55 | −9,13 |

Tabelle II

| Substanz | Noradrenalin-spasmus | Kalium-chlorid-spasmus |
|---|---|---|
| ISDN | $1{,}30 \times 10^{-6}$ | $3{,}10 \times 10^{-6}$ |
| ISMN | $1{,}60 \times 10^{-5}$ | $2{,}40 \times 10^{-6}$ |
| 1c | $1{,}40 \times 10^{-7}$ | $3{,}18 \times 10^{-6}$ |
| 2b | $2{,}80 \times 10^{-7}$ | $3{,}10 \times 10^{-6}$ |
| 4 | $2{,}20 \times 10^{-6}$ | $2{,}80 \times 10^{-6}$ |
| 5b | $6{,}20 \times 10^{-6}$ | $2{,}25 \times 10^{-5}$ |
| 7b | $2{,}00 \times 10^{-5}$ | $4{,}40 \times 10^{-5}$ |

Tabelle III
Blutdruckversuche an Meerschweinchen (i.v.)

| Substanz | Dosis mg/kg | Blutdruck vorher mm Hg | nachher mm Hg | Δ mm Hg |
|---|---|---|---|---|
| ISDN | 0,25 | 68,70±2,30 | 57,00±2,10 | –11,70 |
| | 1,00 | 66,00±3,50 | 46,30±0,90 | –19,70 |
| | 2,50 | 66,70±1,70 | 37,70±0,90 | –29,00 |
| ISMN | 0,25 | 57,60±3,10 | 53,90±3,10 | – 3,70 |
| | 1,00 | 54,70±3,80 | 48,40±3,10 | – 6,30 |
| | 2,50 | 52,30±4,80 | 41,40±3,90 | –10,90 |
| 1c | 0,25 | 61,30±2,60 | 59,70±2,40 | – 1,60 |
| | 1,00 | 61,90±1,90 | 46,90±1,20 | –15,00 |
| | 2,50 | 60,60±1,80 | 37,60±1,00 | –23,00 |
| 2b | 0,25 | 58,70±4,30 | 47,30±2,60 | –11,40 |
| | 1,00 | 58,30±2,00 | 42,70±1,80 | –15,60 |
| | 2,50 | 53,70±3,30 | 33,00±1,00 | –20,70 |
| 5b | 0,25 | 62,70±3,00 | 55,00±1,00 | – 7,70 |
| | 1,00 | 62,30±4,10 | 44,30±0,90 | –18,00 |
| | 2,50 | 61,30±4,90 | 33,30±0,70 | –28,00 |

Tabelle IV

| Substanz + Dosis | Zeit (min) nach Appl. | Frequenz $min^{-1}$ | Blutdruck mm Hg | Δ | dp/dt mm sec. | Δ |
|---|---|---|---|---|---|---|
| ISDN | 0 | 174,3± 9,3 | 104,3± 7,7 | –13,9 | 8 800± 831 | – 800 |
| 5 mg/kg | 10 | 179,3± 7,7 | 90,4±12,5 | –13,1 | 8 000±1 097 | –1 133 |
| | 30 | 177,7± 7,7 | 91,2±10,0 | –10,1 | 7 766± 807 | –1 167 |
| | 60 | 175,0± 8,6 | 94,2± 9,2 | – 9,3 | 7 633± 743 | –1 750 |
| | 120 | 166,7± 9,4 | 95,0± 9,6 | | 7 050± 661 | |
| 5b | 0 | 211,5±19,3 | 123,5± 7,3 | –25,2 | 10 625±2 083 | –2 575 |
| 5 mg/kg | 10 | 209,5±19,2 | 98,3± 6,2 | –28,7 | 8 050±1 204 | –2 650 |
| | 30 | 212,5±21,6 | 94,8± 7,2 | –29,7 | 7 975±1 216 | –2 350 |
| | 60 | 225,0±16,2 | 93,8± 7,8 | –22,2 | 8 275± 309 | –3 125 |
| | 120 | 228,0±15,6 | 101,3±11,2 | | 7 500±1 022 | |

Herzfrequenz ($min^{-1}$), Blutdruck (mm Hg) und Inotropie (mm Hg/sec) nach intraduodenaler Applikation von ISDN oder Substanz Nr. 5b an Katzen.
Mittelwerte ± Standardfehler aus je 6 Tieren.

Zur orientierenden Prüfung der akuten Toxizität einiger der erfindungsgemässen Verbindungen wurden diese als Hydrochloride in physiologischer Kochsalzlösung an weiblichen NMRI-Albinomäusen intravenös in Dosen von 100 bzw. 200 mg/kg appliziert. Die Substanzen wurden an mindestens 3 Tieren pro Dosis gespritzt. Wenn nach der höchsten applizierten Dosis noch kein Tier gestorben war, wurden keine weiteren Substanzdosen geprüft. Im Zweifelsfall wurde die Prüfung an mindestens 3 weiteren Tieren mit der gleichen Dosis wiederholt. Beobachtet wurde die Todesrate innerhalb 24 Stdn. nach der Applikation

In Tabelle V sind die ermittelten Todesraten und der daraus abgeschätzte $LD_{50}$-Bereich dargestellt.

Tabelle V

| Beispiel Nr. | Todeshäufigkeit bei der i.v.-Dosis v. 200 mg/kg | 100 mg/kg | Geschätzter $LD_{50}$-Bereich (mg/kg) |
|---|---|---|---|
| 2b/3b | 2/6 | 0/6 | ≥200 |
| 5b | – | 0/6 | >100 |
| 6/7 | 0/6 | – | >200 |
| 8 | 3/3 | 0/6 | <200, >100 |

**Patentansprüche**

1. 2-O- und 5-O-substituierte 1.4;3.6-Dianhydrohexit-mononitrate der allgemeinen Formel I,

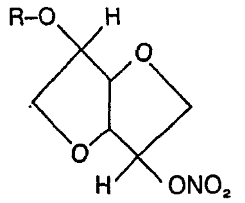

(I)

worin R eine halogensubstituierte Phenylgruppe, eine Methansulfonyl- oder Nicotinoylgruppe ist, sowie deren physiologisch unbedenkliche Säureadditionssalze, sofern R die Nicotinoylgruppe bedeutet.

2. 5-O-substituierte 1.4;3.6-Dianhydro-L-idit-2-nitrate der allgemeinen Formel V,

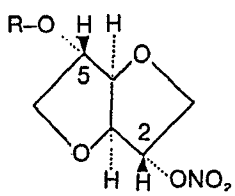

(V)

worin R die in Anspruch 1 angegebenen Bedeutungen besitzt, sowie deren physiologisch unbedenkliche Säureadditionssalze.

3. 5-O-substituierte 1.4;3.6-Dianhydro-D-glucit-2-nitrate der allgemeinen Formel VI,

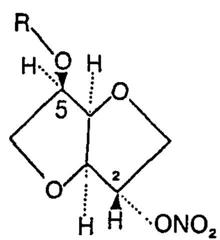

(VI)

worin R die in Anspruch 1 angegebenen Bedeutungen besitzt, sowie deren physiologisch unbedenkliche Säureadditionssalze.

4. 2-O-substituierte 1.4;3.6-Dianhydro-D-glucit-5-nitrate der allgemeinen Formel VII,

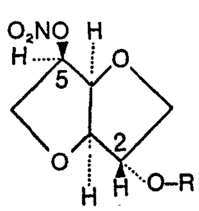

(VII)

worin R die in Anspruch 1 angegebenen Bedeutungen besitzt, sowie deren physiologisch unbedenkliche Säureadditionssalze.

5. 5-O-substituierte 1.4;3.6-Dianhydro-D-mannit-2-nitrate der allgemeinen Formel VIII,

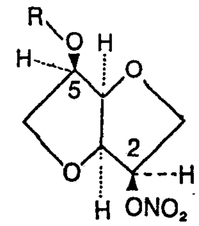

(VIII)

worin R die in Anspruch 1 angegebenen Bedeutungen besitzt, sowie deren physiologisch unbedenkliche Säureadditionssalze.

6. 5-O-(4-Chlorphenyl)-1.4;3.6-dianhydro-L-idit-2-nitrat.

7. 1.4;3.6-Dianhydro-L-idit-2-nitrat-5-nicotinat.

8. 1.4;3.6-Dianhydro-D-glucit-2-nitrat-5-methansulfonat.

9. 1.4;3.6-Dianhydro-D-glucit-2-nitrat-5-nicotinat.

10. 1.4;3.6-Dianhydro-D-glucit-2-nicotinat-5-nitrat.

11. 1.4;3.6-Dianhydro-D-mannit-2-nitrat-5-nicotinat.

12. 1.4;3.6-Dianhydro-D-mannit-2-nitrat-5-methansulfonat.

13. Verfahren zur Herstellung der Verbindungen der allgemeinen Formel I, worin R eine Methansulfonyl- oder Nicotinoylgruppe ist, dadurch gekennzeichnet, dass

(A) L-Isoidid, D-Isosorbid oder D-Isomannid mit Methansulfonsäurechlorid oder Nicotinsäurechlorid in den entsprechenden Mono-O-acyl-1.4;3.6-dianhydro-hexit übergeführt wird,

(B) wonach die freie Hydroxylgruppe des entstandenen Mono-O-acyl-1.4;3.6-dianhydro-hexits oder gegebenenfalls dessen Säureadditionssalzes mit Salpetersäure, Nitriersäure oder mit einem Gemisch aus Salpetersäure und Eisessig/Acetanhydrid, gegebenenfalls in Gegenwart von Harnstoff, zum entsprechenden 2-O- bzw. 5-O-acylierten 1.4;3.6-Dianhydro-hexitmononitrat verestert wird.

14. Verfahren zur Herstellung der Verbindungen der allgemeinen Formel I, worin R eine Methansulfonyl- oder Nicotinoylgruppe ist, dadurch gekennzeichnet, dass

(a) L-Isoidid, D-Isosorbid oder D-Isomannid mit Salpetersäure, Nitriersäure oder mit einem Gemisch aus Salpetersäure und Eisessig/Acetanhydrid, gegebenenfalls in Gegenwart von Harnstoff zum entsprechenden 1.4;3.6-Dianhydrohexit-mononitrat umgesetzt wird,

(b) welches anschliessend mit Methansulfonsäurechlorid oder Nicotinsäurechlorid zum entsprechenden 2-O- bzw. 5-O-acylierten 1.4;3.6-Dianhydrohexit-mononitrat umgesetzt wird.

15. Verfahren zur Herstellung der Verbindungen gemäss Anspruch 1, worin R einen Halogenphenylrest bedeutet, dadurch gekennzeichnet, dass

(A′) L-Isoidid, D-Isosorbid oder D-Isomannid mit einem Sulfonsäurechlorid in den entsprechenden Mono-O-acyl-1.4;3.6-dianhydro-hexit übergeführt wird,

(B′) welcher anschliessend durch Umsetzung mit einem reaktiven Salz eines halogensubstituierten Phenols in Gegenwart eines dipolaren aprotischen Lösungsmittels bei erhöhter

Temperatur unter Konfigurationsumkehr in den entsprechenden 1.4;3.6-Dianhydro-hexithalogenphenyläther übergeführt wird,

(C') wonach letzterer mit Salpetersäure, Nitriersäure oder mit einem Gemisch aus Salpetersäure und Eisessig/Acetanhydrid, gegebenenfalls in Gegenwart von Harnstoff, zum entsprechenden 2-O- bzw. 5-O-substituierten 1.4;3.6-Dianhydrohexit-mononitrat umgesetzt wird.

16. Verfahren nach Anspruch 15, dadurch gekennzeichnet, dass als Sulfonsäurechlorid Methansulfonsäurechlorid oder Toluolsulfonsäurechlorid verwendet wird.

17. Verfahren zur Herstellung der Verbindungen gemäss den Ansprüchen 1 oder 4, worin R die Nicotinoyl-Gruppe bedeutet, dadurch gekennzeichnet, dass D-Isosorbid oder D-Isomannid mit einem Sulfonsäurechlorid in das 1.4;3.6-Dianhydro-D-glucit-5-acylat bzw. 1.4;3.6-Dianhydro-D-mannit-2-acylat übergeführt wird, welches anschliessend durch Umsetzung mit einem geeigneten Nicotinsäuresalz in Gegenwart eines dipolaren aprotischen Lösungsmittels bei erhöhter Temperatur unter Konfigurationsumkehr in das entsprechende 1.4;3.6-Dianhydro-L-idit-5-nicotinat bzw. 1.4;3.6-Dianhydro-D-glucit-2-nicotinat übergeführt wird, wonach das so erhaltene Isohexid-nicotinat mit Salpetersäure, Nitriersäure oder mit einem Gemisch aus Salpetersäure und Acetanhydrid/Essigsäure, gegebenenfalls in Gegenwart von Harnstoff, zum entsprechenden 1.4;3.6-Dianhydro-L-idit-5-nicotinat-2-nitrat bzw. zum 1.4;3.6-Dianhydro-D-glucit-2-nicotinat-5-nitrat verestert wird.

18. Verfahren nach Anspruch 17, dadurch gekennzeichnet, dass als Sulfonsäurechlorid Methansulfonsäurechlorid verwendet wird.

19. Verfahren nach Anspruch 17 oder 18, dadurch gekennzeichnet, dass als Nicotinsäuresalz Natriumnicotinat verwendet wird.

20. Verfahren nach einem der Ansprüche 17 bis 19, dadurch gekennzeichnet, dass als dipolares aprotisches Lösungsmittel Dimethylformamid verwendet wird.

21. Pharmazeutische Zubereitung, enthaltend eine Verbindung gemäss einem der Ansprüche 1 bis 12 sowie übliche Träger und Zusatzstoffe.

**Claims**

1. 2-O- and 5-O-substituted 1.4;3.6-Dianhydrohexitol mononitrates of the general formula I,

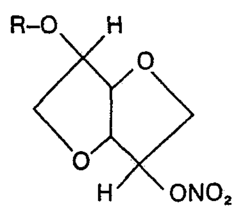

(I)

wherein R is a halogen-substituted phenyl group, a methanesulphonyl or nicotinoyl group, as well as their physiologically acceptable acid-addition salts, insofar as R signifies the nicotinoyl group.

2. 5-O-Substituted 1.4;3.6-dianhydro-L-iditol 2-nitrates of the general formula V,

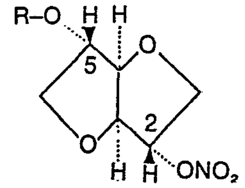

(V)

wherein R possesses the meanings given in claim 1, as well as their physiologically acceptable acid-addition salts.

3. 5-O-Substituted 1.4;3.6-dianhydro-D-glucitol 2-nitrates of the general formula VI,

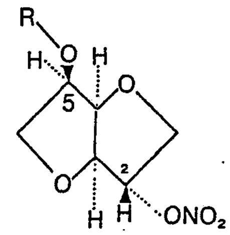

(VI)

wherein R possesses the meanings given in claim 1, as well as their physiologically acceptable acid-addition salts.

4. 2-O-Substituted 1.4;3.6-dianhydro-D-glucitol 5-nitrates of the general formula VII,

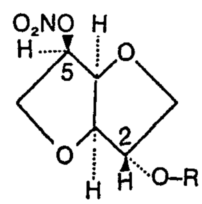

(VII)

wherein R possesses the meanings given in claim 1, as well as their physiologically acceptable acid-addition salt.

5. 5-O-Substituted 1.4;3.6-dianhydro-D-mannitol 1-nitrates of the general formula VIII,

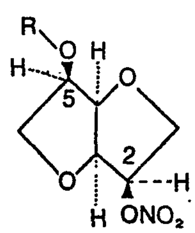

(VIII)

wherein R possesses the meanings given in claim 1, as well as their physiologically acceptable acid-addition salts.

6. 5-O-(4-Chlorophenyl)-1.4;3.6-dianhydro-L-iditol 2-nitrate.

7. 1.4;3.6-Dianhydro-L-iditol 2-nitrate 5-nicotinate.

8. 1.4;3.6-Dianhydro-D-glucitol 2-nitrate 5-methanesulphonate.

9. 1.4;3.6-Dianhydro-D-glucitol 2-nitrate 5-nicotinate.

10. 1.4;3.6-Dianhydro-D-glucitol 2-nicotinate 5-nitrate.

11. 1.4;3.6-Dianhydro-D-mannitol 2-nitrate 5-nicotinate.

12. 1.4;3.6-Dianhydro-D-mannitol 2-nitrate 5-methanesulphonate.

13. Process for the preparation of compounds of general formula I, wherein R is a methanesulphonyl or nicotinoyl group, characterised in that

(A) L-isoidide, D-isosorbide or D-isomannide is converted into the corresponding mono-O-acyl-1.4;3.6-dianhydrohexitol with methanesulphonic acid chloride or nicotinic acid chloride,

(B) whereafter the free hydroxyl group of the resulting mono-O-acyl-1.4;3.6-dianhydrohexitol or possibly its acid-addition salt is esterified with nitric acid, nitrating acid or with a mixture of nictric acid and glacial acetic acid/acetic anhydride, possibly in the presence of urea, to give the corresponding 2-O- or 5-O-acylated 1.4;3.6-dianhydrohexitol mononitrate.

14. Process for the preparation of compounds of general formula I, wherein R is a methanesulphonyl or nicotinoyl group, characterised in that

(a) L-isoidide, D-isosorbide or D-isomannide is reacted with nitric acid, nitrating acid or with a mixture or nitric acid and glacial acetic acid/acetic anhydride, possibly in the presence of urea, to give the corresponding 1.4;3.6-dianhydrohexitol mononitrate,

(b) which is subsequently reacted with methanesulphonic acid chloride or nicotinic acid chloride to give the corresponding 2-O- or 5-O-acylated 1.4;3.6-dianhydrohexitol mononitrate.

15. Process for the preparation of compounds according to claim 1, wherein R signifies a halophenyl radical, caracterised in that

(A') L-isoidide, D-isosorbide or D-isomannide is converted into the corresponding mono-O-acyl-1.4;3.6-dianhydrohexil with a sulphonic acid chloride,

(B') which is subsequently converted into the corresponding 1.4;3.6-dianhydrohexitol halophenyl ether by reaction with a reactive salt of a halogensubstituted phenol in the presence of a dipolar aprotic solvent at an elevated temperature, with reversal of configuration,

(C') whereafter the latter is reacted with nitric acid, nitrating acid or with a mixture of nitric acid and glacial acetic acid/acetic anhydride, possibly in the presence of urea to give the corresponding 2-O- 5-O-substituted 1.4;3.6-dianhydrohexitol mononitrate.

16. Process according to claim 15, characterised in that methanesulphonic acid chloride or toluenesulphonic acid chloride is used as said sulphonic acid chloride.

17. Process for the preparation of compounds according to claims 2 or 4, wherein R signifies the nicotinoyl group, characterised in that D-isosorbide or D-isomannide is converted with a sulphonic acid chloride into the 1.4;3.6-dianhydro-D-glucitol 5-acylate or 1.4;3.6-dianhydro-D-mannitol 2-acylate, which is subsequently converted by reaction with a suitable nicotinic acid salt in the presence of a dipolar aprotic solvent at an elevated temperature, with reversal of configuration, into the corresponding 1.4;3.6-dianhydro-L-iditol 5-nicotinate or 1.4;3.6-dianhydro-D-glucitol 2-nicotinate, whereafter the so obtained isohexide nicotinate is esterified with nitric acid, nitrating acid or with a mixture of nitric acid and acetic anhydride/acetic acid, possibly in the presence of urea, to give the corresponding 1.4,.3.6-dianhydro-L-iditol 5-nicotinate 2-nitrate or to the 1.4;3.6-dianhydro-D-glucitol 2-nicotinate 5-nitrate.

18. Process according to claim 17, characterised in that methanesulphonic acid chloride is used as said sulphonic acid chloride.

19. Process according to claim 17 or 18, characterised in that sodium nicotinate is used as said nicotinic acid salt.

20. Process according to anyone of claims 17 to 19, characterised in that dimethylformamide is used as said dipolar aprotic solvent.

21. Pharmaceutical composition, containing a compound according to anyone of claims 1 to 12, as well as conventional carrier and additive materials.

## Revendications

1. 1.4;3.6-dianhydro-hexite-mononitrates 2-O- et 5-O-substitués de formule générale I,

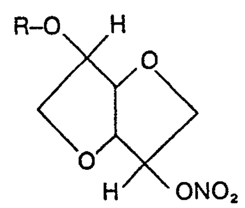

(I)

où R représente un groupe phényle halogénosubstitué, un groupe méthanesulfonyle ou nicotinoyle, ainsi que leurs sels d'addition d'acides physiologiquement acceptables, dans la mesure où R représente le groupe nicotinoyle.

2. 1.4;3.6-dianhydro-L-idite-2-nitrates 5-O-substitués de formule générale V,

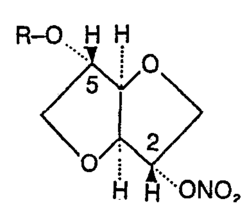

(V)

où R a les significations données dans la revendication 1, ainsi que leurs sels d'addition d'acides physiologiquement acceptables.

3. 1.4;3.6-dianhydro-D-glucite-2-nitrates 5-O-substitués de formule générale VI,

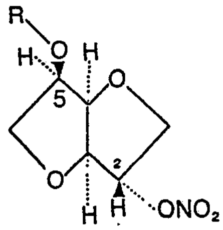

(VI)

où R a les significations données dans la revendication 1, ainsi que leurs sels d'addition d'acides physiologiquement acceptables.

4. 1.4;3.6-dianhydro-D-glucite-5-nitrates 2-O-substitués de formule générale VII,

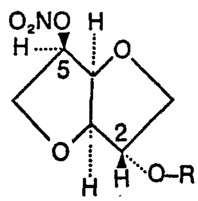

(VII)

où R a les significations données dans la revendication 1, ainsi que leurs sels d'addition d'acides physiologiquement acceptables.

5. 1.4;3.6-dianhydro-D-mannite-2-nitrates 5-O-substitués de formule générale VIII,

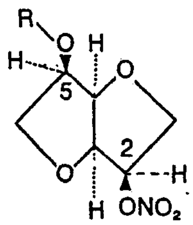

(VIII)

où R a les significations données dans la revendication 1, ainsi que leurs sels d'addition d'acides physiologiquement acceptables..

6. 5-O-(4-chlorophényl)-1.4;3.6-dianhydro-L-idite-2-nitrate.

7. 1.4;3.6-dianhydro-L-idite-2-nitrate-5-nicotinate.

8. 1.4;3.6-dianhydro-D-glucite-2-nitrate-5-méthanesulfonate.

9. 1.4;3.6-dianhydro-D-glucite-2-nitrate-5-nicotinate.

10. 1.4;3.6-dianhydro-D-glucite-2-nicotinate-5-nitrate.

11. 1.4;3.6-dianhydro-D-mannite-2-nitrate-5-nicotinate.

12. 1.4;3.6-dianhydro-D-mannite-2-nitrate-5-méthanesulfonate.

13. Procédé de préparation des composés de formule générale I, où R est un groupe méthanesulfonyle ou nicotinoyle, caractérisé en ce que

(A) on transforme du L-isoidide, du D-isosorbide ou du D-isomannide avec du chlorure de l'acide méthanesulfonique ou du chlorure de l'acide nicotinique en le mono-O-acyl-1.4;3.6-dianhydro-hexite correspondant,

(B) après quoi on estérifie le groupe hydroxyle libre du mono-O-acyl-1.4;3.6-dianhydro-hexite apparu ou éventuellement son sel d'addition d'acide avec l'acide nitrique, un mélange sulfonitrique ou avec un mélange d'acide nitrique et d'acide acétique glacial/acétanhydride, éventuellement en présence d'urée, pour donner le 1.4;3.6-dianhydro-hexite-mononitrate 2-O- ou selon les cas 5-O-acylé correspondant.

14. Procédé de préparation des composés de formule générale I, où R est un groupe méthanesulfonyle ou nicotinoyle, caractérisé en ce que

(a) on fait réagir du L-isoidide, du D-isosorbide ou du D-isomannide avec de l'acide nitrique, un mélange sulfonitrique ou avec un mélange d'acide nitrique et d'acide acétique glacial/acétanhydride, éventuellement en présence d'urée pour donner le 1.4;3.6-dianhydro-hexite-mononitrate correspondant,

(b) qu'ensuite en fait réagir avec du chlorure de l'acide méthanesulfonique ou du chlorure de l'acide nicotinique pour donner le 1.4;3.6-dianhydro-hexite-mononitrate 2-O- ou selon les cas 5-O-acylé correspondant.

15. Procédé de préparation des composés selon la revendication 1, où R représente un radical halogénophényle, caractérisé en ce que

(A') on transforme du L-isoidide, du D-isosorbide ou du D-isomannide avec un chlorure d'acide sulfonique pour donner le mono-O-acyl-1.4;3.6-dianhydro-hexite correspondant,

(B') qu'ensuite on transforme après réaction avec un sel réactif d'un phénol halogéno-substitué en présence d'un solvant aprotique dipolaire à température augmentée avec inversion de configuration en le 1.4;3.6-dianhydro-hexite-halogènephényléther correspondant,

(C') après quoi on fait réagir ce dernier avec de l'acide nitrique, un mélange sulfonitrique ou avec un mélange d'acide nitrique et d'acide acétique glacial/acétanhydride, éventuellement en présence d'urée, pour donner le 1.4;3.6-dianhydro-hexite-mononitrate 2-O- ou selon les cas 5-O-substitué correspondant.

16. Procédé selon la revendication 16, caractérisé en ce qu'on utilise comme chlorure d'acide sulfonique le chlorure de l'acide méthanesulfonique ou le chlorure de l'acide toluènesulfonique.

17. Procédé de préparation des composés selon les revendications 2 ou 4, où R représente le groupe nicotinoyle, caractérisé en ce qu'on transforme le D-isosorbide ou le D-isomannide avec un chlorure d'acide sulfonique en le 1.4;3.6-dianhydro-D-glucite-5-acylate ou selon les cas en le 1.4;3.6-dianhydro-D-mannite-2-acylate, qu'on transforme ensuite par réaction avec un sel d'acide nicotinique approprié en présence d'un solvant aprotique dipolaire à température augmentée avec inversion de configuration en le 1.4;3.6-dianhydro-L-idite-5-nicotinate ou selon les cas le 1.4;3.6-dianhydro-D-glucite-2-nicotinate, après quoi on estérifie l'isohexide-nicotinate ainsi obtenu avec de l'acide nitrique, un mélange sulfonitrique ou avec un mélange d'acide nitrique et d'acétanhydride/acide acétique, éventuellement en présence d'urée, pour donner le 1.4;3.6-dianhydro-L-idite- 5-nicotinate-2-nitrate ou selon les cas le 1.4;3.6-dianhydro-d-glucite-2-nicotinate-5-nitrate correspondant.

18. Procédé selon la revendication 17, caractérisé en ce qu'on utilise comme chlorure d'acide sulfonique le chlorure de l'acide méthanesulfonique.

19. Procédé selon la revendication 17 ou 18, caractérisé en ce qu'on utilise comme sel d'acide nicotinique le nicotinate de sodium.

20. Procédé selon l'une des revendications 17 à 19, caractérisé en ce qu'on utilise comme solvant aprotique dipolaire le diméthylformamide.

21. Préparations pharmaceutiques contenant un composé selon l'une des revendications 1 à 12 ainsi que les supports et additifs habituels.